# EUROPEAN PATENT APPLICATION

(11) **EP 1 872 799 A1**
(43) Date of publication of application: **02.01.2008**
(21) Application number: 06730857.7
(22) Date of filing: 31.03.2006
(51) Int. Cl.: A61K 47/30, A61K 9/70, A61K 47/32, A61K 47/34, A61K 47/36, A61K 47/38, A61K 47/42, A61N 1/30, A61M 35/00

(54) **EXTERNAL PREPARATION, METHOD OF APPLYING EXTERNAL PREPARATION, IONTOPHORESIS DEVICE AND TRANSDERMAL PATCH**

(30) Priority: 15.04.2005 JP 2005119024; 02.08.2005 US 195351
(71) Applicant: Transcu Ltd., 048580 Singapore (SG)
(72) Inventor: MATSUMURA, Akihiko, Sibuya-Ku, Tokyo 150-0022 (JP); NAKAYAMA, Mizuo, Sibuya-Ku, Tokyo 150-0022 (JP); MATSUMURA, Takehiko, Sibuya-Ku, Tokyo 150-0022 (JP); AKIYAMA, Hidero, Sibuya-Ku, Tokyo 150-0022 (JP); TSUJI, Kazuyuki, Sibuya-Ku, Tokyo 150-0022 (JP); SHIBATA, Tsutomu, Sibuya-Ku, Tokyo 150-0022 (JP); TANIOKA, Akihiko, 1450061 (JP); MINAGAWA, Mie, 1080074 (JP)
(74) Representative: Schäfer, Matthias W.
(86) International application number: PCT/JP2006/306909
(87) International publication number: WO 2006/112254

(57) **Abstract**

The administration efficiency of a drug may be improved by establishing compatibility between the suppression of the release of a biological counter ion from a living body's skin and the maintenance of a good state of contact between each of a working electrode structure and a nonworking electrode structure and the skin. Coating films each composed of an external preparation containing: a hydrophilic polymer matrix agent; and an ion-exchange resin dispersed in the hydrophilic polymer matrix agent, or coating films each composed of an external preparation containing a hydrophilic polymer having an ion-exchange function are formed on the skin, and iontophoresis is performed through the coating films.

## Description

### FIELD OF THE INVENTION

The invention relates to an external preparation used for drug administration by means of iontophoresis, a method of applying an external preparation, and an iontophoresis device and a percutaneous patch to be used in combination with the external preparation.

### BACKGROUND OF THE INVENTION

An iontophoresis device generally includes a working electrode structure holding a drug solution whose active ingredients are dissociated to positive or negative drug ions through dissolution and a nonworking electrode structure that functions as a counter electrode of the working electrode structure. The drug ions are administered to a living body by the application of a voltage with the same polarity as that of the drug ions (a voltage of first conductivity type) to the working electrode structure and the application of a voltage with the opposite polarity thereof (a voltage of second conductivity type) to the nonworking electrode structure under the condition that both the structures are in contact with a skin of the living body (human being or animal).

In this case, the charge supplied to the working electrode structure is consumed by the movement of the drug ions to the living body and the release of biological counter ions (ions which are present in the living body and charged in a conductivity type opposite to that of the drug ions) to the working electrode structure side. Typically, the biological counter ions (e.g., Na⁺ and Cl⁻) having a small molecular weight and hence having a large mobility are released mainly from the living body. Hence, the ratio of the charge consumed by the release of biological counter ion increases, which makes it impossible to administer drug ions effectively.

When power is continuously supplied for a certain period of time or longer for drug administration, further problems occur, which may include the occurrence of inflammation considered to result from a change in pH value or in ion balance on the skin surface with which both structures are brought into contact.

Patent documents 1 and 2 disclose iontophoresis devices that have solved the above mentioned problem.

More specifically, in each of the iontophoresis devices disclosed in the patent documents 1 and 2, a working electrode structure is composed of a first electrode, a drug holding part which receives power supply from the first electrode, and an ion-exchange membrane that is placed on a front side of the drug holding part (side toward the skin) and selectively passes ions of the same conductivity type (first conductivity type) as that of the drug ions held by the drug holding part, and the drug ions are administered through the ion-exchange membrane, thereby suppressing the release of biological counter ions from the living body and the occurrence of inflammation on the skin surface in contact with the working electrode structure.

In addition, in the iontophoresis device described in the patent document 2, a nonworking electrode structure is composed of a second electrode, an electrolyte solution holding part that receives power supply from the second electrode, and a second ion-exchange membrane that is placed on a front side of the electrolyte solution holding part (side toward the skin) and selectively passes ions of a conductivity type (second conductivity type) opposite to that of the drug ions, whereby the occurrence of inflammation on the skin surface into which the nonworking electrode is brought into contact is suppressed.

Herein, in the iontophoresis devices described in the patent documents 1 and 2, the working electrode structure of the iontophoresis device has a 5-layer structure including the first electrode, an electrolyte solution holding part for holding an electrolyte solution in contact with the first electrode, an ion-exchange membrane for selectively passing ions having the second conductivity type, the drug holding part and the ion-exchange membrane for selectively passing ions having the first conductivity type, or further, the nonworking electrode structure of the iontophoresis device has a 5-layer structure including the second electrode, an electrolyte solution holding part in contact with the second electrode, an ion-exchange membrane for selectively passing ions having the first conductivity type, the electrolyte solution holding part, and the ion-exchange membrane for selectively passing ions having the second conductivity type. As a result, the additional effects such as prevention of the drug ions from being decomposed in the vicinity of the electrode member and prevention of the movement of H⁺ or OH⁻ ions generated at the first and second electrodes to the skin interface of a living body are achieved.

In the iontophoresis devices of the patent documents 1 and 2, in order to facilitate the passage of drug ions with a relatively large molecular weight and effectively suppressing the release of biological counter ions from a living body, an ion-exchange membrane is used in which a porous film made of polyolefin, vinyl chloride-based resin, fluorine-based resin, or the like is filled with ion-exchange resin. However, such a porous film has low affinity for the skin of a living body, and it is difficult to keep the (electrical or physical) contact between the ion-exchange membrane and the skin of the living body in a satisfactory state during the administration of drug ions. Depending upon the site with which a working electrode structure and a nonworking electrode structure are brought into contact, the behavior of the living body (patient) during the administration, and the like, the administration efficiency of drug ions cannot be maintained at a sufficient level.

Therefore, the following inconvenience is caused. During the administration of the drug ions, it is necessary to interpose an electrolyte solution or the like between the ion-exchange membrane and the skin of the living body, or further keep pressing the working electrode structure and the nonworking electrode structure against the skin of the living body with some bias means.

Further, when an electrolyte is interposed between the ion-exchange membrane and the skin, the function of the ion-exchange membrane of suppressing the release of biological counter ions from the skin degrades. Consequently, even when the electrolyte is interposed in such a manner, the drug administration cannot be performed in an ideal state.

Patent document 3 discloses a technique in which physiologically active peptide is prevented from adsorbing in a hardly redissoluble manner to the skin surface by cleaning the skin surface with, for example, an aqueous solution containing a cation surfactant at the time of percutaneous administration of the physiologically active peptide by means of iontophoresis, to thereby improve the controllabilityofdose. However, the cation surfactant in the patent document 3 does not correspond to an ion-exchange resin or a hydrophilic polymer having an ion-exchange function. Moreover, the patent document 3 does not suggest a combination of the cation surfactant and administration of either one of positively or negatively charged drug ion.
Patent Document 1: JP 3030517 B
Patent Document 2: JP 2000-229128 A
Patent Document 3: JP 08-163212 A

### DISCLOSURE OF INVENTION

### PROBLEMS TO BE SOLVED

The object of the present invention is to further enhance the administration efficiency of a drug by realizing suppression of the release of biological counter ion from a living body and also maintenance of a satisfactory contact state between a working electrode structure and a skin.

Another object of the present invention is to enable safe and efficient drug administration by alleviating damage such as inflammation generated at the skin with which a working electrode structure or a nonworking electrode structure are brought into contact and making the contact states of both the structures with the living body's skin good.

Still another object of the present invention is to suppress a decomposition of a drug ion in the vicinity of the electrode and to suppress the change in pH at the skin interface, to thereby further improve the safety and stability of the administration of a drug.

### MEANS FOR SOLVING PROBLEMS

According to a first invention, there is provided an external preparation containing: a hydrophilic polymer matrix agent; and an ion-exchange resin dispersed in the hydrophilic polymer matrix agent and the above mentioned problem is solved by administrating a drug by means of iontophoresis from a skin (includes mucous membrane) of a living body to which such an external preparation is applied.

Herein, the ion-exchange resin in the first invention is obtained by introducing an ion exchange group whose counter ion is the first conductivity type, into a polymer having a three-dimensional network structure.

More specifically, the external preparation according to the first invention is used in such a manner that, when a drug (the term "drug" in the specification refers to a substance having a physiological effect irrespective of whether preparation or the like in accordance with applications is performed) is administered by means of iontophoresis, power is supplied in a state where an electrode structure (a working electrode structure or a nonworking electrode structure) of an iontophoresis device is brought into contact with the skin to which the external preparation is applied. Used as an external preparation to be applied to the skin with which a positive electrode structure is brought into contact is an external preparation containing, as the ion-exchange resin, a cation exchange resin into which a cation exchange group such as a sulfonic acid group or a carboxylic acid group is introduced. Used as an external preparation to be applied to the skin with which a negative electrode structure is brought into contact is an external preparation containing, as the ion-exchange resin, an anion exchange resin into which an anion exchange groups such as quaternary ammonium or primary to tertiary ammonium is introduced.

The hydrophilic polymer matrix agent in the first invention is a polymer serving as a binder for maintaining an appropriate dispersed state of an ion-exchange resin in an external preparation and having some degree or more of solubility or swelling property with respect to an aqueous solvent (such as water, glycerin, polyethylene glycol, ethyl alcohol, or propanol). From the viewpoint of, for example, safety for a living body, a hydrophilic polymer matrix agent made of polyvinyl alcohol, collagen, sericin, polyethylene oxide, chitin, chitosan, sucrose, gelatin, hyaluronic acid, alginic acid, fibroin, polylactic acid, gum arabic, agar, sodium alginate, polyvinyl pyrrolidone, carbopol, methylcellulose, hydroxypropylcellulose, hydroxypropylmethylcellulose, carmellose, carmellose sodium, carmellose calcium, or hydroxyapatite, or a mixture thereof can preferably be used.

The external preparation according to the first invention is blended with a solvent such as water, ethyl alcohol, or propanol for adjusting the viscosity of the external preparation to an appropriate one.

According to a second invention, there is provided an external preparation for iontophoresis, containing a hydrophilic polymer having an ion-exchange function. The above problem is solved by performing drug administration by means of iontophoresis from the living body's skin (including mucous membrane) to which such an external preparation is applied.

The hydrophilic polymer in the external preparation according to the second invention can be a cellulose-based resin (such as regenerated cellulose, cellulose ester, cellulose ether, or nitrocellulose), collagen, sericin, chitin, chitosan, gelatin, hyaluronic acid, alginic acid, fibroin, or polylactic acid, or a mixture thereof. In the case of such an external preparation as well, an appropriate solvent such as water, ethyl alcohol, or propanol is blended for imparting an appropriate viscosity to the external preparation.

To be specific, the external preparation according to the second invention is used in such a manner that, when a drug is administered by means of iontophoresis, power is supplied in a state where an electrode structure of an iontophoresis device is brought into contact with the skin to which the external preparation according to the second invention is applied. Used as an external preparation to be applied to the skin with which an positive electrode structure is brought into contact is an external preparation containing, as the hydrophilic polymer having a cation-exchange function, a cellulose-based resin, hyaluronicacid, alginic acid, or polylactic acid, or a mixture thereof. If a pH value during drug administration is maintained at an isoelectric point or higher, an external preparation containing, as the hydrophilic polymer having a cation-exchange function, collagen, sericin, or fibroin, or a mixture thereof may also be used.

Used as an external preparation to be applied to the skin with which a negative electrode structure is brought into contact is an external preparation containing, as the hydrophilic polymer having an anion-exchange function, chitin or chitosan, or a mixture thereof. If a pH value during drug administration is maintained at an isoelectric point or lower, an external preparation containing, as the hydrophilic polymer having an anion-exchange function, collagen, sericin, gelatin, or fibroin, or a mixture thereof may also be used.

The external preparations of the first and second inventions can be accordingly blended with additional components such as a moisturizing agent, an anti-inflammatory agent, a perfume, a coloring agent, a viscosity modifier, a stabilizing agent, a surfactant, a plasticizer, a solubilizing agent, a buffering agent, a base agent, an adsorbent, a binder, a suspending agent, an anti-oxidant, an antifoamer, a tonicity agent, a pH regulator, an emulsifer, an adhesive enhancement agent, a high-density gum, a dispersant, a fragrance, a preservative, a solvent, a solubilizer, a solubilizing agent, and a plasticizer in addition to the above-mentioned component.

In addition, the external preparation according to the first or second invention is preferably applied uniformly to a living body's skin in such a manner that a coating film of the applied external preparation has as small a thickness as possible (for example, several micrometers to several hundred of micrometers) to such an extent that the coating film can maintain a function as an ion-exchange membrane (function of selectively passing a positive or negative ion) during drug administration by means of iontophoresis. To this end, the external preparation according to the first or second invention of the present invention is preferably applied to the living body's skin by means of electrostatic coating.

According to a third invention, there is provided an iontophoresis device, including a working electrode structure including:
a first electrode;
a first electrolyte solution holding part in contact with the first electrode;
a first ion-exchange membrane for selectively passing an ion of a second conductivity type, the first ion-exchange membrane being placed on a front side of the first electrolyte solution holding part;
a drug holding part for holding a drug solution containing drug ions of a first conductivity type, the drug holding part being placed on a front side of the first ion-exchange membrane; and
a first liner attached to a surface on a front side of the drug holding part, the first liner being removed upon administration of the drug ions into a living body.

Such an iontophoresis device is preferably used in combination with the external preparation according to any one of the appended claims 1 to 7. In storing and handling the iontophoresis device, the first liner prevents the drying of the drug holding part and the contamination of foreign matter into the drug holding part. In addition, a drug can be administered by applying a voltage of a first conductivity type to the first electrode in a state where the exposed drug holding part (by removing the first liner) is brought into direct contact with a coating film composed of the external preparation according to any one of the claims 1 to 7.

In this case, the coating film composed of the external preparation according to any one of the claims 1 to 7 acts as an ion-exchange membrane for selectively passing an ion of the first conductivity type. Therefore, the release of a biological counter ion from the living body' s skin may be suppressed. At the same time, states of contact between the skin and the coating film and contact between the coating film and the drug holding part are kept good by virtue of an effect of the hydrophilic polymer matrix agent or the hydrophilic polymer in the coating film, whereby a drastic improvement in administration efficiency of the drug is achieved.

A filmy body made of an arbitrary material (such as a resin film or a metal film) which is capable of preventing the evaporation of a water content in the drug holding part and the contamination of foreign matter into the drug holding part and which has a strength with which the filmy body is not readily broken during the storage and handling of the iontophoresis device can be used as the first liner in the device.

In addition, in the iontophoresis device, the decomposition of a drug ion in the first electrolyte solution holding part and a change in pH value in the drug holding part are suppressed by the first ion-exchange membrane arranged between the first electrolyte solution holding part and the drug holding part. As a result, sufficient safety and stability in drug administration are secured.

The iontophoresis device according to the third invention may further include a nonworking electrode structure including:
a second electrode;
a second electrolyte solution holding part in contact with the second electrode;
a second ion-exchange membrane for selectively passing ions of the first conductivity type, the second ion-exchange membrane being placed on a front side of the second electrolyte solution holding part;
a third electrolyte solution holding part placed on a front side of the second ion-exchange membrane; and
a second liner attached to a front side of the third electrolyte solution holding part, the second liner being removed upon administration of the drug ion into a living body.

In storing and handling the iontophoresis device, the second liner prevents the drying and alteration of the third electrolyte solution holding part and the contamination of foreign matter into the third electrolyte solution holding part. In addition, a drug can be administered by applying a voltage of the second conductivity type to the second electrode in a state where the exposed third electrolyte solution holding part by removing the second liner is brought into direct contact with a coating film composed of the external preparation according to any one of the claims 1 to 7.

In this case, the coating film composed of the external preparation according to any one of the claims 1 to 7 with which the third electrolyte solution holding part is brought into contact acts as an ion-exchange membrane for selectively passing an ion of the second conductivity type. Therefore, damage to the skin caused by power supply is alleviated. At the same time, states of contact between the skin and the coating film and contact between the coating film and the third electrolyte solution holding part are kept good by virtue of an effect of the hydrophilic polymer matrix agent or the hydrophilic polymer in the coating film, whereby an additional improvement in administration efficiency of the drug or an improvement in safety of drug administration is achieved.

A filmy body made of an arbitrary material (such as a resin film or a metal film) which is capable of preventing the evaporation of a water content in the third electrolyte solution holding part and the contamination of foreign matter into the third electrolyte solution holding part and which has a strength with which the filmy body is not readily broken during the storage and handling of the iontophoresis device can be used as the second liner in the device.

In addition, in the iontophoresis device, a change in pH value in the third electrolyte solution holding part is suppressed by the second ion-exchange membrane arranged between the second electrolyte solution holding part and the third electrolyte solution holding part. As a result, sufficient safety and stability in drug administration are secured.

According to a fourth invention, there is provided an iontophoresis device, including a working electrode structure including:
a first electrode; and
a drug holding part for holding a drug solution containing drug ions of a first conductivity type, the drug holding part receiving power from the first electrode, wherein the drug ions are administered by applying a voltage of the first conductivity type to the first electrode in a state where the drug holding part is brought into contact with a coating film formed on a skin, the coating film being composed of an external preparation comprising: a hydrophilic polymer matrix agent; and an ion-exchange resin dispersed in the hydrophilic polymer matrix agent, the ion-exchange resin being introduced with an ion exchange group whose counter ion is the first conductivity type, or a hydrophilic polymer having a function of exchanging an ion of the first conductivity type.

In the fourth invention, the external preparation applied to the skin functions as an ion-exchange membrane for selectively passing ions of the first conductivity type, because the external preparation contains a hydrophilic polymer matrix agent and an ion-exchange resin which is dispersed in the hydrophilic polymer matrix agent and is introduced with an ion exchange group whose counter ion is the first conductivity type, or a hydrophilic polymer having a function of exchanging ions of the first conductivity type. As a result, drug ions can be administered to a living body while the release of a biological counter ion from the skin is suppressed. At the same time, the state of contact between the living body's skin and the coating film of the external preparation or contact between the coating film of the external preparation and the drug holding part is kept good by virtue of the effect of the hydrophilic polymer matrix agent or the hydrophilic polymer in the coating film of the external preparation, whereby a drastic improvement in administration efficiency of the drug is achieved.

In the fourth invention, the iontophoresis device may further include a nonworking electrode structure including:
a second electrode; and
a third electrolyte solution holding part that receives power supply from the second electrode, and
the drug ions may be administered by applying a voltage of the second conductivity type to the second electrode in a state where the third electrolyte solution holding part is brought into contact with a coating film formed on the skin, the coating film being composed of an external preparation containing: a hydrophilic polymer matrix agent; and an ion-exchange resin dispersed in the hydrophilic polymer matrix agent, the ion-exchange resin being introduced an ion exchange group whose counter ion is the second conductivity type, or a hydrophilic polymer having a function of exchanging an ion of the second conductivity type.

In this case, the external preparation applied to the skin functions as an ion-exchange membrane for selectively passing an ion of the second conductivity type, because the external preparation contains a hydrophilic polymer matrix agent and an ion-exchange resin which is dispersed in the hydrophilic polymer matrix agent and is introduced with an ion exchange group whose counter ion is the second conductivity type, or a hydrophilic polymer having a function of exchanging an ion of the second conductivity type. Therefore, damage to the skin caused by power supply may be alleviated. At the same time, the state of contact between the living body's skin and the coating film of the external preparation or contact between the coating film of the external preparation and the third electrolyte solution holding part is kept good by virtue of the effect of the hydrophilic polymer matrix agent or the hydrophilic polymer in the coating film of the external preparation.

In the iontophoresis device according to the fourth invention, the working electrode structure may further include: a first electrolyte solution holding part holding an electrolyte solution in contact with the first electrode; and a first ion-exchange membrane for selectively passing ions of the second conductivity type, the first ion-exchange membrane being arranged between the first electrolyte solution holding part and the drug holding part. In this case, the drug holding part receives power from the first electrode through the first electrolyte solution holding part and the first ion-exchange membrane. With this configuration, a change in pH value in the drug holding part can be suppressed, and safety and stability in drug administration can be further improved.

In the iontophoresis device according to the fourth invention, the nonworking electrode structure may further include: a second electrolyte solution holding part holding an electrolyte solution in contact with the second electrode; and a second ion-exchange membrane for selectively passing ions of the first conductivity type, the second ion-exchange membrane being arranged between the second electrolyte solution holding part and the third electrolyte solution holding part. In this case, the third electrolyte solution holding part receives power from the second electrode through the second electrolyte solution holding part and the secondion-exchange membrane. With this configuration, a change in pH value in the third electrolyte solution holding part can be suppressed, and safety and stability in drug administration can be further improved.

According to a fifth invention, there is provided a percutaneous patch, including a drug holding part for holding a drug solution containing drug ions charged to a first conductivity type, in which the drug ions are administered to a living body by bringing the drug holding part into contact with a coating film formed on a skin, the coating film being composed of an external preparation comprising: a hydrophilic polymer matrix agent; and an ion-exchange resin dispersed in the hydrophilic polymer matrix agent, the ion-exchange resin being introduced with an ion exchange group whose counter ion is the first conductivity type, or a hydrophilic polymer having a function of exchanging an ion of the first conductivity type.

In the fifth invention, the external preparation applied to the skin functions as an ion-exchange membrane for selectively passing ions of the first conductivity type, because the external preparation comprises: a hydrophilic polymer matrix agent; and an ion-exchange resin which is dispersed in the hydrophilic polymer matrix agent and is introduced with an ion exchange group whose counter ion is the first conductivity type, or a hydrophilic polymer having a function of exchanging an ion of the first conductivity type. Therefore, the movement of an ion of the second conductivity type (drug counter ion) present in the drug holding part into a living body or the movement of a biological counter ion from the living body into the drug holding part is suppressed. As a result, mixing of a drug ion in the drug holding part and an ion of the first conductivity type present in the living body is promoted, whereby administration efficiency of the drug ion can be increased.

### BREIF DESCRIPTION OF THE DRAWINGS

[Figure 1] Figure 1 is a schematic view showing an iontophoresis device according to the present invention for administering a drug whose active ingredient is dissociated to positive ions.
[Figure 2] Figure 2 is a schematic view showing an iontophoresis device according to the present invention for administering a drug whose active ingredient is dissociated to negative ions.
[Figure 3] Figure 3 is a schematic view showing an iontophoresis device according to the present invention for administering a drug whose active ingredient is dissociated to positive ions.
[Figure 4] Figure 4 is a schematic view showing an iontophoresis device according to the present invention for administering a drug whose active ingredient is dissociated to negative ions.
[Figure 5] Figure 5 is a schematic view showing how a drug is percutaneously administered according to the present invention.
[Figure 6] Figure 6 is a schematic view showing how a drug is percutaneously administered according to the present invention.
[Figure 7] (a) and (b) are explanatory views each showing a configuration of a percutaneous patch according to an embodiment of the present invention and how the percutaneous patch is used.
[Figure 8] is an' explanatory view showing an exemplary configuration of an electrostatic coating machine that can be used for coating an external preparation of the present invention.

### BEST MODE FOR CARRYING OUT THE INVENTION

Hereinafter, the embodiment of the present invention is explained.

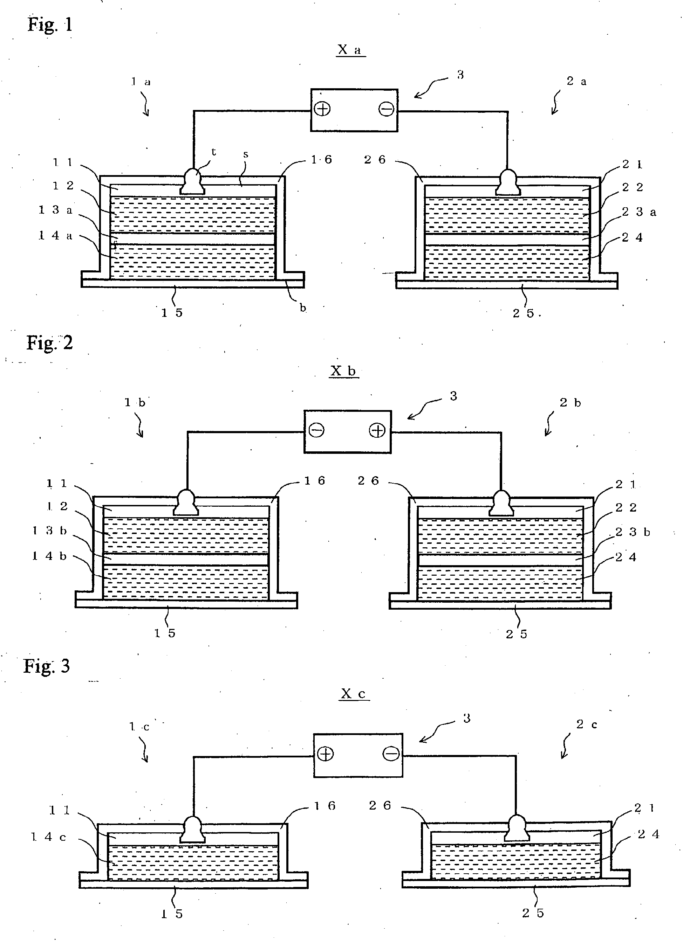

Figure 1 is an explanatory view showing a basic configuration of an iontophoresis device Xa according to the present invention for administering a drug whose active ingredient is dissociated to positive ions (such as lidocaine as an anesthetic agent or morphine hydrochloride as an anesthetic agent).

As shown in the drawing, the iontophoresis device Xa of the present invention includes as main components (members) a working electrode structure 1a, a nonworking electrode structure 2a, and a power source 3.

The working electrode structure 1a includes an electrode member 11 connected to a positive pole of the power source 3, an electrolyte solution holding part 12 for holding an electrolyte solution in contact with the electrode member 11, an anion exchange membrane 13a placed on a front side of the electrolyte solution holding part 12, a drug holding part 14a placed on a front side of the anion exchange membrane 13a and supplied with power from the electrode member 11 through the electrode solution holding part 12 and the anion exchange membrane 13a, and a liner 15 placed on the front side of the drug holding part 14a. The entire working electrode structure 1a is housed in a cover or a container 16.

On the other hand, the nonworking electrode structure 2a includes an electrode member 21 connected to a negative pole of the power source 3, an electrolyte solution holding part 22 for holding an electrolyte solution in contact with the electrode member 21, a cation exchange membrane 23a placed on a front side of the electrolyte solution holding part 22, an electrolyte solution holding part 24 placed on a front side of the cation exchange membrane 23a and supplied with power from the electrode member 21 through the electrolyte solution holding part 22' and the anion exchange membrane 23a, and a liner 25 placed on a front side of the electrolyte solution holding part 24. The entire nonworking electrode structure 2a is housed in a cover or a container 26.

In the iontophoresis device Xa, an electrode made of an arbitrary conductive material may be used for each of the electrode members 11 and 21 without limitation. An active electrode such as a silver/silver chloride couple electrode capable of suppressing the generation of H⁺ and OH⁻ ions due to electrolysis of water may also be used. In the iontophoresis device Xa, however, the electrolyte solution of each of the'electrolyte solution holding parts 12 and 22 may be blended with a substance having oxidation/reduction potential lower than that of water to suppress the generation of a gas, and the electrolyte solution may also be prepared as a buffer containing multiple kinds of ions, so fluctuation of pH can be suppressed. In this case, an inactive electrode made of carbon, platinum, or the like may also be used without any problem.

However, a composite carbon electrode 11 or 21 composed of: a terminal member (t) obtained by blending a polymer matrix with carbon powder; and a conductive sheet (s) made of carbon fibers or carbon fiber paper is particularly preferably used. With this electrode in which high conductivity and flexibility is compatible, the administration efficiency of a drug can be increased without degrading adhesiveness between each of the structures 1a and 2a and a skin, and the movement of a metal ion eluted from an electrode member into a living body can be prevented.

Furthermore, the electrolyte solution holding parts 12, 22, and 24 in the iontophoresis device Xa hold an electrolyte solution so as to keep the conductivity. Phosphate buffered saline, physiological saline, etc. can be used as the electrolyte solution typically.

Furthermore, in order to more effectively prevent the generation of a gas caused by the electrolytic reaction of water and the increase in a conductive resistance caused by the generation of gas, or the change in pH caused by the electrolytic reaction of water, an electrolyte that is more readily oxidized or reduced (oxidation at the positive pole and the reduction at the negative pole) than the electrolytic reaction of water can be added to the electrolyte solution holding parts 12 and 22. In terms of the biological safety and economic efficiency (low cost and easy availability), for example, an inorganic compound such as ferrous sulfate or ferric sulfate, a medical agent such as ascorbic acid (vitamin C) or sodium ascorbate, and an organic acid such as lactic acid, oxalic acid, malic acid, succinic acid, or fumaric acid and/or a salt thereof can be used preferably. Alternatively, a combination of those substances (for example, 1:1 mixed aqueous solution containing 1 mol (M) of lactic acid and 1 mol (M) of sodium fumarate) can also be used.

The electrolyte solution holding parts 12, 22, and 24 may hold the above-mentioned electrolyte solution in a liquid state. However, the electrolyte solution holding parts 12, 22, and 24 may be configured by impregnating or infiltrating a carrier made of any material having water holding property, such as: fabric sheet such as gauze or filter paper; or polymer gel sheet such as hydrogel of acrylic resin (acrylic hydrogel) or segmented polyurethane gel with the above-mentioned electrolyte solution, thereby enhancing the ease of handling thereof.

The drug holding part 14a in the iontophoresis device Xa according to this embodiment holds an aqueous solution of a drug (for example, lidocaine or morphine hydrochloride) whose active ingredient is dissociated to positive drug ions by the dissolution, as a drug solution.

Herein, the drug holding part 14a may hold a drug solution in a liquid state. However, the drug holding part 14a may be configured by impregnating or infiltrating a carrier made of any material having water holding property, such as: fabric sheet such as gauze or filter paper; or polymer gel sheet such as hydrogel of acrylic resin (acrylic hydrogel) or segmented polyurethane gel with the drug solution, thereby enhancing the ease of handling thereof.

When such a carrier is used as the electrolyte solution holding part 12, 22 or 24, or the drug holding part 14a, an appropriate impregnation rate or content should be set to obtain a sufficient conductivity or transport number. By appropriately setting the impregnation rate or content of the drug, a high transport number (high drug delivery property), e.g., 70% can be obtained in the drug holding part 14a.

The impregnation ratio or content in the present specification is represented by % by weight (i. e., 100 x (W-D)/D[%] where D is a weight in a dry state and W is a weight after impregnation) . The transport number is a ratio of a current contributing to the drug ion movement with respect to all the current supplied to the working electrode structure.

The anion-exchange membranes 13a that can be used in the iontophoresis device Xa include an arbitrary anion-exchange membrane having a function of selectively passing anions therethrough, such as NEOSEPTAs (AM-1, AM-3, AMX, AHA, ACH, ACS, and so on), manufactured by Tokuyama Co., Ltd. Among them, an anion-exchange membrane that includes a porous film, a portion of or whole pores of which is filled with an ion-exchange resin having an anion-exchange function is used preferably..

The cation-exchange membranes 23a that can be used include an arbitrary cation-exchange membrane having a function of selectively passing cations therethrough, such as NEOSEPTAs (CM-1, CM-2, CMX, CMS, CMB, and so on), manufactured by Tokuyama Co., Ltd. Among them, a cation-exchange membrane that includes a porous film having pores, a portion of or whole pores of which is filled with an ion-exchange resin having a cation-exchange function is used preferably.

Herein, a fluorine type resin with an ion-exchange group introduced to a perfluorocarbon skeleton or a hydrocarbon type resin containing a resin that is not fluorinated as a skeleton can be used as the above-mentioned ion-exchange resin. In view of the convenience of a production process, a hydrocarbon type ion-exchange resin is preferable. Although the filling ratio of the ion-exchange resin is also related to the porosity of the porous film, the filling ratio is generally 5 to 95% by mass, in particular, approximately 10 to 90% by mass, and 20 to 60% by mass is preferred.

The ion-exchange group in the above-mentioned ion-exchange resin is not particularly limited so far as it is a functional group that generates a group having a negative or positive charge in aqueous solutions. Specific examples of the functional group that can serve as such an ion-exchange group include cation exchange groups such as a sulfonic acid group, a carboxylic acid group, and a phosphonic acid group. These acid groups can be present as free acids or in the form of salts. Counter cations for the salts of the acids include alkali metal cations such as sodium ion and potassium ion, and ammonium ion. Among these cation-exchange groups, generally, a sulfonic acid group, which is a strong acid group, is particularly preferred. The anion-exchange groups include, for example, a primary amino group, a secondary amino group, a tertiary amino group, a quaternary ammonium group, a pyridyl group, an imidazole group, a quaternary pyridinium group, and a quaternary imidazolium group. Counter anions for these anion-exchange groups include halogen ions such as chlorine ion, hydroxy ion, and so on. Among these anion-exchange groups, generally a quaternary ammonium group and a quaternary pyridinium group, which are strong basic groups, is preferred.

The above-mentioned porous film is not particularly limited and any porous film can be used as far as it is in the form of a film or a sheet that has a lot of pores communicating both sides thereof. To satisfy both of high strength and flexibility, it is preferable that the porous film be made of a thermoplastic resin.

Examples of the thermoplastic resins constituting the porous film include, without limitation: polyolefin resins such as homopolymers or copolymers of α-olefins such as ethylene, propylene, 1-butene, 1-pentene, 1-hexene, 3-methyl-1-butene, 4-methyl-1-pentene, and 5-methyl-1-heptene; vinyl chloride resins such as polyvinyl chloride, vinyl chloride-vinyl acetate copolymers, vinyl chloride-vinylidene chloride copolymers, and vinyl chloride-olefin copolymers; fluorine resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 6 and nylon 66; and those which are made from polyamide resins. Polyolefin resins are preferably used as they are superior in mechanical strength, flexibility, chemical stability, and chemical resistance, and have good compatibility with ion-exchange resins. As the polyolefin resins, polyethylene and polypropylene are particularly preferable and polyethylene is most preferable.

The physical properties of the above-mentioned porous film made of the thermoplastic resin are not particularly limited. However, it is preferable that the pore has a mean pore size of preferably 0.005 µm to 5.0 µm, more preferably 0.01 µm to 2.0 µ m, most preferably 0.02 µ m to 0.2 µm because ion exchange membranes that are thin and have excellent strengths and low electric resistances can be readily obtained. The above-mentioned mean pore size as used herein means a mean flow pore size measured by the bubble point method according to JIS K3832-1990. A porosity of the porous film is preferably 20 to 95%, more preferably 30 to 90%, most preferably 30 to 60%. The thickness of the porous film is preferably 5 µm to 140 µm, more preferably 10 µm to 120 µm, most preferably 15 µm to 55 µm. Usually, anion-exchange membranes and cation-exchange membranes that include such porous films have the same thickness as that of the porous film or up to about 20 □m larger than the thickness of the porous film.

Each of the liners 15 and 25 in the iontophoresis device Xa is intended for preventing the evaporation of a drug solution or of an electrolyte solution and the contamination of foreign matter into these solutions by being attached to a surface on a front side of each of the drug solution holding part 14a and the electrolyte solution holding part 24. A filmy body made of an arbitrary material (such as a resin film or a metal film) which is capable of suppressing permeation of water, which has a strength with which the filmy body is not readily broken during the storage and handling of the iontophoresis device Xa, and which can be easily removed at the time of use of the iontophoresis device Xa (administration of a drug) can be used as each of the liners.

Each of the covers or containers 16 and 26 in the iontophoresis device Xa is intended for: preventing the leak or evaporation of an electrolyte solution or a drug solution from each of the electrolyte solution holding parts 12, 22, and 24, and the drug holding part 14a; preventing the contamination of foreign matter from the outside; or imparting, to each of the structures 1a and 1b, such a strength that no problem occurs during, for example, handling of the structure. A cover or container of an arbitrary material (such as a plastic or a metal), a shape, and dimensions capable of achieving such an object can be used.

At the lower end portions (b) of the covers or containers 16 and 26, it is also possible to provide adhesive layers for enhancing adhesiveness to the liners 15 and 25 or the skin (or the coating film composed of the external preparation provided on the skin).

A battery, a voltage stabilizer, a current stabilizer, a voltage/current stabilizer, or the like can be used as the power source 3 in the iontophoresis device Xa. It is preferable to use a current stabilizer that is operated under safe voltage conditions in which an arbitrary current can be adjusted in a range of 0.01 to 1.0 mA/cm2, preferably 0.01 to 0.5 mA/cm2, specifically, at 50 V or less, preferably, 30 V or less.

Figure 2 is a schematic explanatory view showing a configuration of an iontophoresis device Xb according to the present invention for administering a drug whose active ingredient is dissociated to negative ions (such as ascorbic acid as a vitamin agent).

As shown in Figure 2, the iontophoresis device Xb is different from the iontophoresis device Xa in that: the electrode member 11 is connected to the negative terminal of the power source 3 and the electrode member 21 is connected to the positive terminal of the power source 3; and a cation exchange membrane 13b, a drug holding part 14b, and an anion exchange membrane 23b are arranged instead of the anion exchange membrane 13a, the drug holding part 14a, and the cation exchange membrane 23a in the iontophoresis device Xa. Otherwise, the iontophoresis device Xb has the same configuration as that of the iontophoresis device Xa.

In addition, the cation exchange membrane 13b and the anion exchange membrane 23b may be the same as those used for the cation exchange membrane 23a and the anion exchange membrane 13a, respectively. The drug holding part 14b may have the same configuration as that of the drug holding part 14a except that an aqueous solution of a drug, such as ascorbic acid, whose active ingredient is dissociated to negative ions is dissociated.

Figure 3 is a schematic view showing an iontophoresis device Xc according to the present invention for administering a drug whose active ingredient is dissociated to positive ions.

The iontophoresis device Xc includes: a working electrode structure 1c composed of an electrode member 11 connected to a positive pole of the power source 3, a drug holding part 14c which is in contact with the electrode member 11 so that power is directly supplied therefrom, a liner 15 attached to the front surface of the drug holding part 14c, and a cover or a container 16 for storing them; a nonworking electrode structure 2c composed of an electrode member 21 connected to a negative pole of the power source 3, an electrolyte solution holding part is in contact with the electrode member 21 to be directly energized thereby, a liner 25 attached to the front surface of the electrolyte solution holding part 24, and a cover or a container 26 for storing them. The power source 3, the electrode members 11 and 21, the electrolyte solution holding part 24, the liners 15 and 25, and the covers or containers 16 and 26 each have the same configuration as that of the corresponding member in the iontophoresis device Xa. The drug holding part 14c has the same configuration as that of the drug holding part 14a in the iontophoresis device Xa.

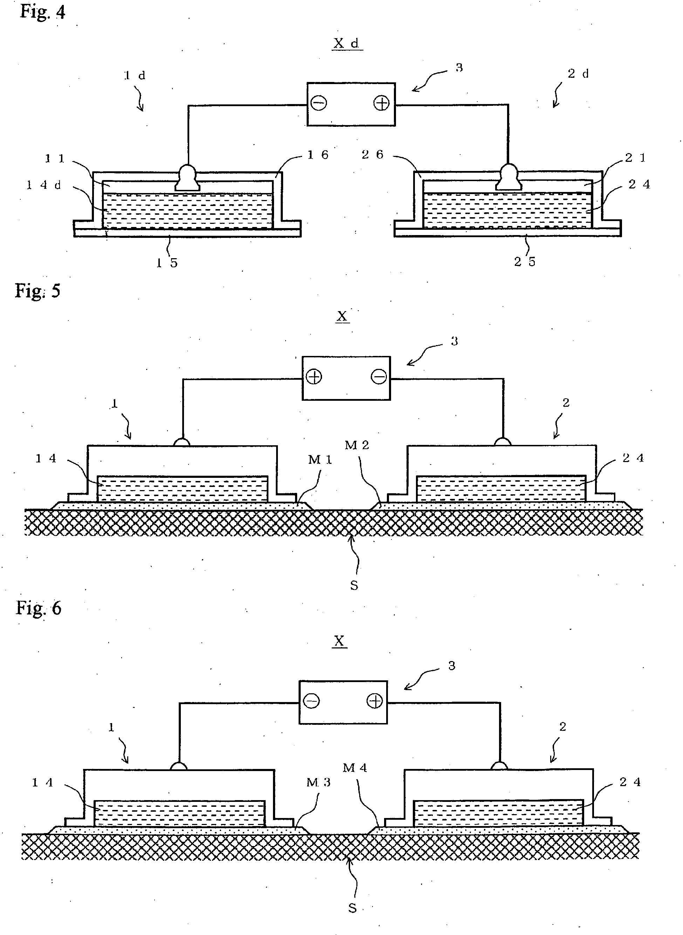

Figure 4 is a schematic view showing an iontophoresis device Xd according to the present invention for administering a drug whose active ingredient is dissociated to negative ions.

The iontophoresis device Xd has the same configuration as that of the iontophoresis device Xc except that a drug holding part 14d is arranged instead of the drug holding part 14c. The drug holding part 14d has the same configuration as that of the drug holding part 14b except that an aqueous solution of a drug, such as ascorbic acid, whose active ingredient is dissociated to negative ions is dissociated.

Figure 5 is a schematic view showing how a drug is percutaneously administered by means of the iontophoresis device Xa or Xc. In the figure, the electrode member 11 or 21, the electrolyte solution holding part 12 or 22, and the ion-exchange membrane 13a or 23a are omitted or in simplification shown. The iontophoresis device Xa or Xc, the working electrode structure 1a or 1c, the nonworking electrode structure 2a or 2c, and the drug holding part 14a or 14c are represented by reference symbols X, 1, 2, and 14, respectively.

In the figure, reference symbol S denotes the skin (or mucous membrane) of a living body to which a drug is administered. Coating films M1 and M2 of an external preparation according to the present invention are formed on the skin S.

The external preparation used for each of the coating films M1 and M2 has a composition composed of: an ion-exchange resin; a hydrophilic polymer matrix agent for maintaining an appropriate dispersed state of the ion-exchange resin; and a solvent such as water, ethyl alcohol, or propanol for adjusting the viscosity of the external preparation to an appropriate one.

Used as the ion-exchange resin for the coating film M1 is an ion-exchange resin obtained by introducing a cation exchange group (an exchange group whose counter ion is cation) such as a sulfonic acid group, a carboxylic acid group, or a phosphonic acid group into a polymer having a three-dimensional network structure such as a hydrocarbon-based resin (for example, a polystyrene resin or an acrylic resin) or a fluorine-based resin having a perfluorocarbon skeleton. The hydrophilic polymer matrix agent is a polymer having a cation-exchange function, having no ion-exchange function, or having an anion-exchange function to such an extent that the ion-exchange function of an ion-exchange resin is not substantially lost, the hydrophilic polymer matrix agent being soluble or being capable of swelling in an aqueous solvent such as water, ethyl alcohol, or propanol. Specific examples thereof include polyvinyl alcohol, collagen, sericin, polyethylene oxide, chitin, chitosan, sucrose, gelatin, hyaluronic acid, alginic acid, fibroin, polylactic acid, gum arabic, agar, sodium alginate, polyvinyl pyrrolidone, carbopol, methylcellulose, hydroxypropylcellulose, hydroxypropylmethylcellulose, carmellose, carmellose sodium, carmellose calcium, and hydroxyapatite, and mixtures thereof.

Used as the ion-exchange resin for the coating film M2 is an ion-exchange resin obtained by introducing an anion exchange group (exchange groups whose counter ion is anion) such as primary to tertiary amino groups, a quaternary ammonium group, a pyridyl group, an imidazole group, a quaternary pyridinium group, and a quaternary imidazolium group into a polymer having a three-dimensional network structure such as a hydrocarbon-based resin (for example, a polystyrene resin or an acrylic resin) or a fluorine-based resin having a perfluorocarbon skeleton. The hydrophilic polymer matrix' agent is a polymer having an anion-exchange function, having no ion-exchange function, or having a cation-exchange function to such an extent that the ion-exchange function of an ion-exchange resin is not substantially lost, the hydrophilic polymer matrix agent being soluble or being capable of swelling in an aqueous solvent such as water, ethyl alcohol, or propanol. Specific examples thereof include polyvinyl alcohol, collagen, sericin, polyethylene oxide, chitin, chitosan, sucrose, gelatin, hyaluronic acid, alginic acid, fibroin, polylactic acid, gum arabic, agar, sodium alginate, polyvinyl pyrrolidone, carbopol, methylcellulose, hydroxypropylcellulose, hydroxypropylmethylcellulose, carmellose, carmellose sodium, carmellose calcium, and hydroxyapatite, and mixtures thereof.

In addition', as shown in Figure 5, the liner 15 or 25 is removed from the iontophoresis device X at the time when a drug is administered, and the drug holding part 14 and the electrolyte solution holding part 24 are arranged so as to be in direct contact with the coating film M1 and the coating film M2, respectively.

With this arrangement, the coating film M1 functions as a cation exchange membrane for selectively passing a cation by virtue of the effect of an ion-exchange resin in the external preparation of the present invention, into which a cation exchange group is introduced. Therefore, when power is supplied from the power source 3 in this state, drug ions are administered from the drug holding part 14 into the skin S through the coating film M1 by the positive voltage applied from the electrode member 11, while the movement of negative ions (biological counter ion) from the skin S into the drug holding part 14 is prevented.

Similarly, in the nonworking electrode structure 2, the coating film M2 functions as an anion exchange membrane for selectively passing an anion by virtue of the effect of an ion-exchange resin in the external preparation of the present invention, into which an anion exchange group is introduced. Therefore, negative ions in the electrolyte solution holding part 24 move into the skin S through the coating film M2, whereby a required quantity of power supply is secured. At the same time, the occurrence of inflammation on the surface the skin S considered to result from a change in pH value or in ion balance is suppressed.

The hydrophilic polymer matrix agent to be incorporated into each of the coating films M1 and M2, that is, any one of polyvinyl alcohol, collagen, sericin, polyethylene oxide, chitin, chitosan, sucrose, gelatin, hyaluronic acid, alginic acid, fibroin, polylactic acid, gum arabic, agar, sodium alginate, polyvinyl pyrrolidone, carbopol, methylcellulose, hydroxypropylcellulose, hydroxypropylmethylcellulose, carmellose, carmellose sodium, carmellose calcium, and hydroxyapatite, and mixtures thereof, is soluble or can swell in an aqueous solvent. Therefore, the hydrophilic polymer matrix agent has an excellent affinity for each of the skin S, the drug holding part 14, and the electrolyte solution holding part 24. As a result, an interface between the skin S and each of the coating films M1 and M2, an interface between the coating film M1 and the drug holding part 14, and an interface between the coating film M2 and the electrolyte solution holding part 24 each can easily maintain a good adhesion state.

When the iontophoresis device Xc is used in the case shown in Figure 5, phenomena such as: the decomposition of a drug ion in the electrode member 11; and the movement of H⁺ and OH⁻ ions generated at the electrode members 11 and 21 to the interfaces of the skin S may occur. However, when the iontophoresis device Xa is used, those phenomena can be effectively prevented by virtue of actions of the anion exchange membrane 13a and the cation exchange membrane 23a.

In addition, the external preparation for the coating film M1 in Figure 5 may contain, instead of the above composition, a cellulose-based resin, hyaluronic acid, alginic acid, or polylactic acid, or a mixture thereof as a hydrophilic polymer component. Alternatively, if a pH value during drug administration can be maintained at an isoelectric point or higher, it may contain collagen, sericin, gelatin, or fibroin, or a mixture thereof as a hydrophilic polymer component. In addition, the external preparation of the coating film M1 may further contain an appropriate amount of a solvent component, such as water, ethyl alcohol, or propanol, for adjusting the viscosity of the external preparation to an appropriate one. The external preparation for the coating film M2 may contain, instead of the above composition, chitin or chitosan, or a mixture thereof, as a hydrophilic polymer component. Alternatively, if a pH value during drug administration can be maintained at an isoelectric point or lower, it may contain collagen, sericin, gelatin, or fibroin, or a mixture thereof. In addition, the external preparation of the coating filmM2 may further contain an appropriate amount of a solvent component, such as water, ethyl alcohol, or propanol, for adjusting the viscosity of the external preparation to an appropriate one. The same effect as that in the case described above can be achieved in this case as well.

Figure 6 is a schematic view, showing how a drug is percutaneously administered by means of the iontophoresis device Xb or Xd. In the figure, the electrode member 11 or 21, the electrolyte solution holding part 12 or 22, and the ion-exchange membrane 13b or 23b are omitted or shown in simplification. The iontophoresis device Xb or Xd, the working electrode structure 1b or 1d, the nonworking electrode structure 2b or 2d, and the drug holding part 14b or 14d are represented by reference symbols X, 1, 2, and 14, respectively.

As shown in the figure, coating films M3 andM4 of the external preparation according to the present invention are formed on the skin (or mucous membrane) S.

The same external preparation as that described above with respect to the coating film M2 is used for the coating film M3 in this case, while the same external preparation as that described above with respect to the coating film M1 is used for the coating film M4.

In addition, in the same manner as that in the case of Figure 5, the liner 15 or 25 is removed from the iontophoresis device X at the time when a drug is administered, and the drug holding part 14 and the electrolyte solution holding part 24 are arranged so as to be in direct contact with the coating film M3 and the coating film M4, respectively.

With this arrangement, the coating film M3 functions as an anion exchange membrane for selectively passing an anion by virtue of the effect of an ion-exchange resin in the external preparation of the present invention, into which an anion exchange group is introduced. Therefore, when power is supplied from the power source 3 in this state, drug ions are administered from the drug holding part 14 into the skin S through the coating film M3 by the negative electrical voltage applied from the electrode member 11, while the movement of positive ions (biological counter ion) from the skin S into the drug holding part 14 is prevented.

Similarly, in the nonworking electrode structure 2, the coating film M4 functions as a cation exchange membrane for selectively passing a cation by virtue of the effect of an ion-exchange resin in the external preparation of the present invention, into which a cation exchange group is introduced. Therefore, positive ions in the electrolyte solution holding part 24 move into the skin S through the coating film M4, whereby a required quantity of power supply is secured. Meanwhile, the occurrence of inflammation on the surface the skin S considered to result from a change in pH value or in ion balance is suppressed.

In addition, similarly to the coating films M1 and M2 in Figure 5, the coating films M3 and M4 each have an excellent affinity for each of the skin S, the drug holding part 14, and the electrolyte solution holding part 24. As a result, an interface between the skin S and each of the coating films M3 and M4, an interface between the coating film M3 and the drug holding part 14, and an interface between the coating film M4 and the electrolyte solution holding part 24 each can easily maintain a good adhesion state.

When the iontophoresis device Xd is used in the case shown in Figure 6, phenomena such as: the decomposition of drug ions near the electrode member 11; and the movement of H⁺ and OH⁻ ions generated at the electrode members 11 and 21 to the interfaces may occur. However, when the iontophoresis device Xb is used, those phenomena can be effectively prevented by virtue of actions of the cation exchange membrane 13b and the anion exchange membrane 23b.

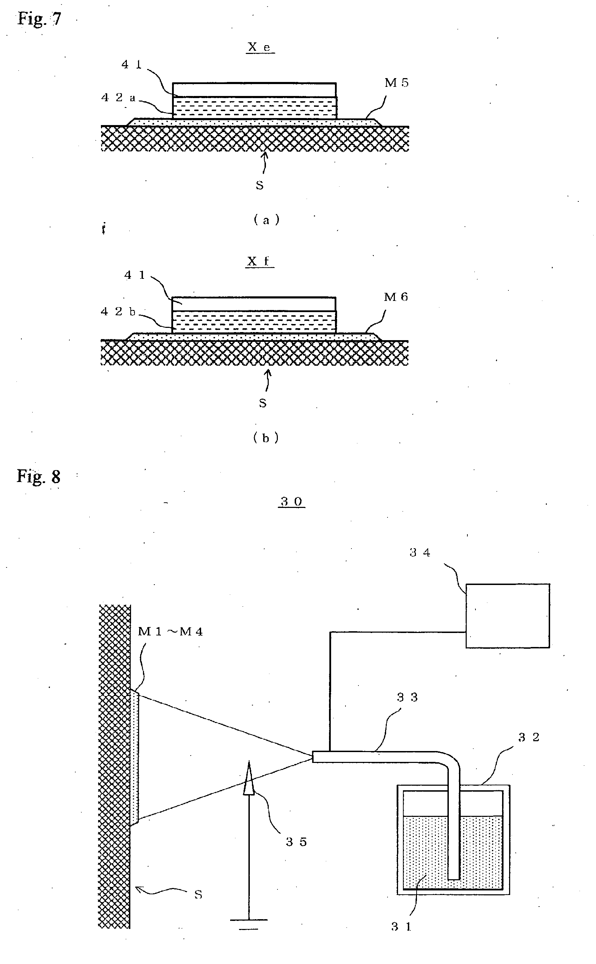

Figures 7(a) (b) show the configurations of percutanoues patches Xe and Xf according to the present invention and how they are used. Figure 7 (a) shows the percutaneous patch Xe for administering a drug whose active ingredient is dissociated to positive ions (e. g., lidocaine as an anesthetic agent or morphine hydrochloride as an anesthetic agent), while Figure 7 (b) shows the percutaneous patch Xf for administering a drug whose active ingredient is dissociated to negative ions (e.g., ascorbic acid as a vitamin agent).

As shown in the figures, the percutaneous patch Xe has a backing material 41 formed of soft plastic such as polyester and a drug holding part 42a for holding an aqueous solution of a drug whose active ingredient is dissociated to positive ions by dissolution. The percutaneous patch Xf has a similar backing material 41 and a drug holding part 42b for holding an aqueous solution of a drug whose active ingredient is dissociated to negative ions by dissolution.

The configurations of the drug holding parts 42a and 42b may be the same as those of the drug holding parts 14a and 14b described above with respect to the iontophoresis devices Xa and Xb.

In addition, the percutaneous patches Xe and Xf are used in such a manner that the drug holding parts 42a and 42b are brought into contact with coating films M5 and M6 of the external preparation of the present invention applied to the skin.

Here, the coating film M5 may be the same as each of the coating films M1 and M4 described above with respect to the iontophoresis devices Xa to Xd, while the coating film M6 may be the same as each of the coating films M2 and M3 described above with respect to the iontophoresis devices Xa to Xd.

In the percutaneous patch Xe, the coating film M5 functions as a cation exchange membrane in the same manner as in each of the coating films M1 and M4. Therefore, the movement of a negative ion (drug counter ion) present in the drug holding part 42a into a living body or the movement of a negatively charged biological counter ion from the living body into the drug holding part 42a is suppressed. As a result, mixing of a drug ion in the drug holding part and a positively charged ion present in the living body is promoted, whereby administration efficiency of the drug ion into the living body can be increased.

Similarly, in the percutaneous patch Xf, the coating film M6 functions as a cation exchange membrane in the same manner as in each of the coating films M2 and M3. Therefore, the movement of a positive ions (drug counter ion) present in the drug holding part 42b into a living body or the movement of a positively charged biological counter ions from the living body into the drug holding part 42b is suppressed. As a result, mixing of drug ions in the drug holding part and negatively charged ions present in the living body is promoted, whereby administration efficiency of the drug ions into the living body can be increased.

It is necessary for the coating films M1 to M6 prepared by the external preparation of the present invention to maintain a function as an ion-exchange membrane for selectively passing a cation or an anion during administration of a drug by means of the iontophoresis devices Xa to Xd or the percutaneous patches Xe and Xf. To this end, the thickness of each of the coating films M1 to M6 necessary for maintaining such a function can easily be determined experimentally in accordance with conditions such as the composition of the external preparation used for each of the coating films and the water content in each of the drug holding parts 14 and 42 and the electrolyte solution holding part 24. In addition, for performing smooth drug administration, each of the coating films M1 to M6 preferably contains an appropriate amount of water at the start of the drug administration. The water content in each of the coating films M1 and M6 can be determined by, for example, the amount of a solvent to be used for the external preparation or conditions for drying after application of the external preparation in accordance with, for example, the composition of the external preparation or the water content in each of the drug holding parts 14 and 42 and the electrolyte solution holding part 24.

Figure 8 is an explanatory view showing an exemplary configuration of an electrostatic coating machine 30 that can be used for coating the external preparation of the present invention.

As shown in the figure, the electrostatic coating machine 30 includes: a tank 32 for storing an external preparation 31 having a composition corresponding to each of the coating films M1 to M4; a coating gun 33 made of a hollow metal pipe having one end immersed in the external preparation in the tank 32; a voltage source 34 for applying a high voltage to the coating gun 33; and an induction electrode 35. The external preparation in the tank can be sprayed to the skin S in mist form by a voltage to be applied by the voltage source 34 between the coating gun 33 and the induction electrode 35.

The use of the electrostatic coating machine 30 enables the external preparation of the present invention to be formed into a coating film having a uniform thickness in the range of, for examples, several micrometers to several hundred micrometers. A coating film having a desired thickness can be formed with high controllability in accordance with, for example, the composition of the external preparation and the water content in each of the drug holding part and the electrolyte solution holding part.

The present invention has been described on the basis of several embodiments. However, the present invention is not limited to those embodiments, and various modifications can be made within the scope of claims.

For example, in each of the above embodiments, the case where an iontophoresis device having no ion-exchange membrane on a front side of the drug holding part (14a to 14d) or of the third electrolyte solution holding part (24) has been described. However, an iontophoresis device having a membrane with selective permeability such as an ion-exchange membrane or an ultrafilter on the front side of at least one of the drug holding part and the third electrolyte solution holding part may be used in combination with the external preparation of the present invention.

That is, when a positive drug ion is administered, an iontophoresis device having: a cation exchange membrane on the front side of a drug holding part in a working electrode structure; and an anion exchange membrane on the front side of a third electrolyte solution holding part in a nonworking electrode structure is used. The cation exchange membrane in the working electrode structure is brought into contact with a coating film of the external preparation of the present invention that functions as a cation exchange membrane applied to skin. The anion exchange membrane in the nonworking electrode structure is brought into contact with a coating film of the external preparation of the present invention that functions as an anion exchange membrane applied to the skin. Thus, a drug can be administered. In such a case as well, a drug ion is administered while the release of a biological counter ion is suppressed. Adhesiveness between the skin and the coating film of the external preparation of the present invention can be kept at a level identical or comparable to that in each of the above embodiments. In addition, adhesiveness between the coating film of the external preparation of the present invention and the cation exchange membrane in the working electrode structure or the anion exchange membrane in the nonworking electrode structure is identical to or better than that in the case of drug administration according to the prior art where no coating film of the external preparation of the present invention is used. This holds true for the case where a negative drug ion is administered, and an external preparation used for such a case is also included in the scope of the present invention..

## Claims

1. An external preparation, comprising:
a hydrophilic polymer matrix agent; and
an ion-exchange resin dispersed in the hydrophilic polymer matrix agent.

2. The external preparation according to claim 1, wherein the hydrophilic polymer matrix agent comprises one of polyvinyl alcohol, collagen, sericin, polyethylene oxide, chitin, chitosan, sucrose, gelatin, hyaluronic acid, alginic acid, fibroin, polylactic acid, gum arabic, agar, sodium alginate, polyvinyl pyrrolidone, carbopol, methylcellulose, hydroxypropylcellulose, hydroxypropylmethylcellulose, carmellose, carmellose sodium, carmellose calcium, and hydroxyapatite, and a mixture thereof.

3. The external preparation according to any one of claims 1 and 2, wherein the ion-exchange resin is obtained by introducing one of a cation exchange group and an anion exchange group into a polymer having a three-dimensional network structure.

4. The external preparation according to claim 3, wherein the cation exchange group comprises one of a sulfonic acid group, a carboxylic acid group, and a phosphonic acid group.

5. The external preparation according to claim 3, wherein the anion exchange group comprises one of primary to tertiary amino groups, a quaternary ammonium group, a pyridyl group, an imidazole group, a quaternary pyridinium group, and a quaternary imidazolium group.

6. An external preparation for iontophoresis, comprising a hydrophilic polymer having an ion-exchange function.

7. The external preparation for iontophoresis according to claim 6, wherein the hydrophilic polymer comprises one of a cellulose-based resin, collagen, sericin, polyethylene oxide, chitin, chitosan, sucrose, gelatin, hyaluronic acid, alginic acid, fibroin, polylactic acid, and hydroxyapatite, and a mixture thereof.

8. A method of applying an external preparation, comprising applying the external preparation to a living body's skin by means of electrostatic coating, wherein the external preparation comprises: a hydrophilic polymer matrix agent; and an ion-exchange resin dispersed in the hydrophilic polymer matrix agent, or a hydrophilic polymer having an ion-exchange function

9. An iontophoresis device, comprising a working electrode structure comprising:
a first electrode;
a first electrolyte solution holding part for holding an electrolyte solution in contact with the first electrode;
a first ion-exchange membrane for selectively passing ions of a second conductivity type, the first ion-exchange membrane being placed on a front side of the first electrolyte solution holding part;
a drug holding part for holding a drug solution containing drug ions of a first conductivity type, the drug holding part being placed on a front side of the first ion-exchange membrane; and
a first liner attached to a surface on a front side of the drug holding part, the first liner being removed upon administration of the drug ion into a living body.

10. The iontophoresis device according to claim 9, further comprising a nonworking electrode structure comprising:
a second electrode;
a second electrolyte solution holding part for holding an electrolyte solution in contact with the second electrode;
a second ion-exchange membrane for selectively passing ions of the first conductivity type, the second ion-exchange membrane being placed on a front side of the second electrolyte solution holding part;
a third electrolyte solution holding part for holding an electrolyte solution, the third electrolyte solution holding part being placed on a front side of the second ion-exchange membrane; and
a second liner attached to a surface on a front side of the third electrolyte solution holding part, the second liner being removed upon administration of the drug ion into a living body.

11. An iontophoresis device, comprising a working electrode structure comprising:
a first electrode; and
a drug holding part for holding a drug solution containing drug ions of a first conductivity type, the drug holding part receiving power supply from the first electrode, wherein the drug ions are administered by applying a voltage of the first conductivity type to the first electrode in a state where the drug holding part is brought into contact with a coating film formed on a skin, the coating film being composed of an external preparation containing: a hydrophilic polymer matrix agent; and an ion-exchange resin dispersed in the hydrophilic polymer matrix agent, the ion-exchange resin being introduced with an ion exchange group whose counter ion is of the first conductivity type, or an external preparation containing a hydrophilic polymer having a function of exchanging an ion of the first conductivity type.

12. The iontophoresis device according to claim 11, further comprising a nonworking electrode structure comprising:
a second electrode; and
an electrolyte solution holding part for holding an electrolyte solution, the electrolyte solution holding part receiving power supply from the second electrode, wherein the drug ions are administered by applying a voltage of the second conductivity type to the second electrode in a state where the electrolyte solution holding part is brought into contact with a coating film formed on a skin, the coating film being composed of an external preparation containing: a hydrophilic polymer matrix agent; and an ion-exchange resin dispersed in the hydrophilic polymer matrix agent, the ion-exchange resin being introduced with an ion exchange group whose counter ion is of the second conductivity type, or a hydrophilic polymer having a function of exchanging an ion of the second conductivity type.

13. A percutaneous patch, comprising:
a drug holding part for holding a drug solution containing drug ions charged to a first conductivity type, wherein the drug ions are administered to a living body by bringing the drug holding part into contact with a coating film formed on a skin, the coating film being composed of an external preparation containing: a hydrophilic polymer matrix agent; and an ion-exchange resin dispersed in the hydrophilic polymer matrix agent, the ion-exchange resin being introduced with an ion-exchange group whose counter ion is of the first conductivity type, or a hydrophilic polymer having a function of exchanging an ion of the first conductivity type.
